(19) Europäisches Patentamt
European Patent Office
Office européen des brevets

(11) Numéro de publication: 0 320 361
A1

# (12) DEMANDE DE BREVET EUROPEEN

(21) Numéro de dépôt: 88403078.4

(22) Date de dépôt: 06.12.88

(51) Int. Cl.4: C 07 D 281/10
C 07 D 417/12, A 61 K 31/55

(30) Priorité: 08.12.87 FR 8717044

(43) Date de publication de la demande:
14.06.89 Bulletin 89/24

(84) Etats contractants désignés:
AT BE CH DE ES FR GB GR IT LI LU NL SE

(71) Demandeur: SYNTHELABO
58 rue de la Glacière
F-75013 Paris (FR)

(72) Inventeur: Lochead, Alistair
142, rue Saint Maur
F-75011 Paris (FR)

Muller, Jean-Claude
4, Avenue de l'Espérance
F-91390 Morsang S/Orge (FR)

Hoornaert, Christian
103, rue Didot
F-75014 Paris (FR)

Denys, Colombe
7, rue André Theuriet
F-92340 Bourg la Reine (FR)

(74) Mandataire: Ludwig, Jacques et al
SYNTHELABO Service Brevets 58, rue de la Glacière
F-75621 Paris Cedex 13 (FR)

Revendications pour les Etats contractants suivants: ES + GR.

(54) **Dérivés de hydroxy-3 (méthoxy-4 phényl)-2 (méthylamino-2 éthyl)-5 dihydro-2,3 5H-benzothiazépine-1,5 one-4, leur préparation et leur application en thérapeutique.**

(57) Composés répondant à la formule générale (I)

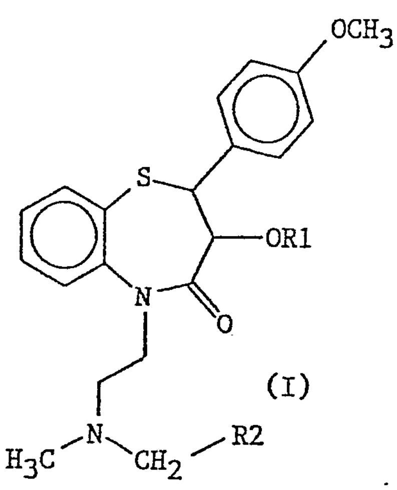

(I)

dans laquelle R1 représente un atome d'hydrogène ou un groupe alcanoyle en $C_2$-$C_4$, et R2 représente un groupe phényle portant èventuellement de 1 à 3 substituants choisis parmi les atomes d'halogène et les groupes méthylènedioxy, éthylènedioxy, et alcoxy en $C_1$-$C_4$,
ainsi que leurs sels d'addition à des acides acceptables en pharmacologie.

Application en thérapeutique.



Bundesdruckerei Berlin

## Description

# DERIVES DE HYDROXY-3 (METHOXY-4 PHENYL)-2 (METHYLAMINO-2 ETHYL)-5 DIHYDRO-2,3 5H-BENZOTHIAZEPINE-1,5 ONE-4, LEUR PREPARATION ET LEUR APPLICATION EN THERAPEUTIQUE

La présente invention a pour objet des dérivés de hydroxy-3 (méthoxy-4 phényl)-2 (méthylamino-2 éthyl)-5 dihydro-2,3 5H-benzothiazépine-1,5 one-4, leur préparation et leur application en thérapeutique.

Les composés de l'invention répondent à la formule générale (I) donnée dans le schéma ci-après, formule dans laquelle

R1 représente un atome d'hydrogène ou un groupe alcanoyle en $C_2$-$C_4$,

R2 représente un groupe phényle portant éventuellement de 1 à 3 substituants choisis parmi les atomes d'halogène et les groupes méthylènedioxy, éthylènedioxy, et alcoxy en $C_1$-$C_4$.

Les composés de formule générale (I) peuvent se présenter à l'état de bases libres ou de sels d'addition à des acides. Les atomes de carbone aux positions 2 et 3 étant asymétriques, ils peuvent aussi exister sous diverses formes diastéréoisomères, racémiques ou optiquement pures. Ces diverses formes font partie de l'invention.

Conformément à l'invention on peut préparer les composés de formule générale (I) selon le schéma donné ci-après.

Une première méthode consiste à faire réagir une benzothiazépinone de formule (VI) (dans laquelle R1 est tel que défini ci-dessus) avec une amine halogénée de formule (V) (dans laquelle R2 est tel que défini ci-dessus et X représente un atome d'halogène tel que le chlore ou le brome).

La seconde méthode consiste à faire réagir une benzothiazépinone de formule (VIII) (dans laquelle R1 est tel que défini ci-dessus) avec un dérivé halogéné de formule (III) (dans laquelle R2 est tel que défini ci-dessus et X représente un atome d'halogène tel que le chlore ou le brome).

Ces deux réactions, entre une amine et un dérivé halogéné, sont classiques, et se déroulent donc dans des conditions bien connues de l'homme du métier, c'est-à-dire par exemple dans un solvant aprotique, tel que l'acétone ou la butanone-2, à la température du reflux, en présence d'une base destinée à fixer l'acide libéré, par exemple le carbonate de potassium, et éventuellement en présence d'un catalyseur de

SCHEMA

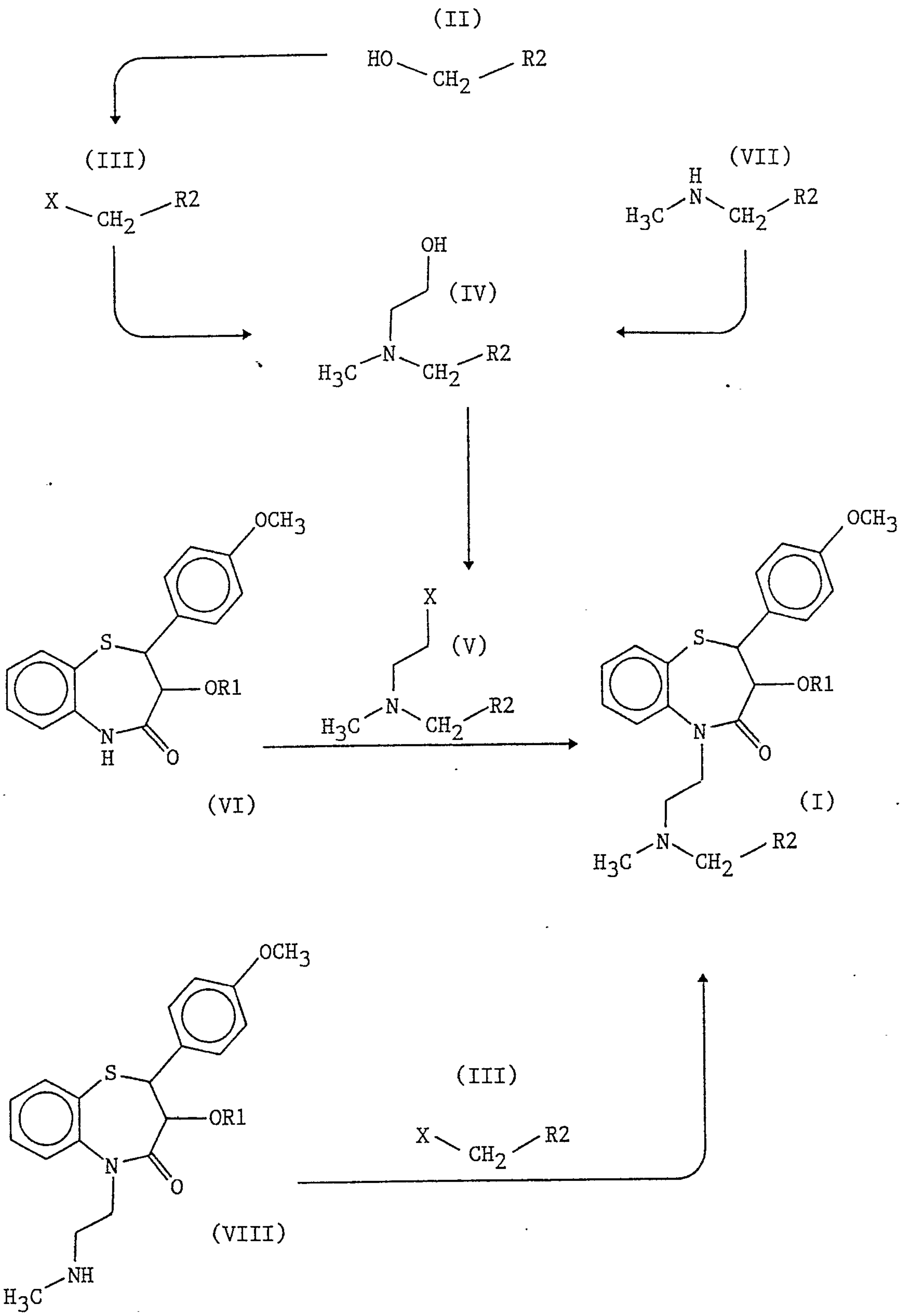

(II)

(III)

(VII)

(IV)

(V)

(VI)

(I)

(VIII)

(III)

transfert de phase tel que l'iodure de tétra-n-butylammonium. Il va de soi que l'on peut transformer un

composé de formule (I) dans laquelle R1 représente un atome d'hydrogène en un composé de formule (I) dans laquelle R1 représente un groupe alcanoyle par une acylation de type connu, et que la transformation inverse est possible par hydrolyse.

Les benzothiazépinones de formules (VI) sont décrites par exemple dans Chem. Pharm. Bull., 18, 2281 (1970), Chem. Pharm. Bull., 19, 595 (1971) et Helv. Chim. Acta, 67, 916 (1984). Les benzothiazépinones de formules (VIII) sont décrites par exemple dans Chem. Pharm. Bull., 26, 2889, (1978), J. Pharmacobio. Dyn., 7, 24 (1978) et J. Cardiov. Pharmacol., 7, 152 (1984).

L'amine halogénée de formule (V) peut être préparée par exemple à partir de l'alcool correspondant, de formule (IV), par action d'un agent d'halogénation tel que le chlorure de thionyle. L'aminoalcool de formule (IV) peut lui même être préparé selon toute méthode connue, par exemple à partir de l'amine de formule (VII), traitée avec le chloro-2 éthanol, ou encore à partir d'un dérivé halogéné de formule (III), traité avec le méthylamino-2 éthanol.

Les composés de formules (III) et (VII) sont décrits dans la littérature et sont pour la plupart disponibles dans le commerce. Les composés de formule (III) non commerciaux sont facilement accessibles à partir des alcools de formule (II).

L'exemple qui va suivre illustre en détail la préparation d'un composé conforme à l'invention.

Les microanalyses élémentaires et les spectres IR et RMN confirment les structures des produits obtenus.

Exemple.

(+)-cis-Acétyloxy-3 (méthoxy-4 phényl)-2 [[N-[(méthylènedioxy-3,4 phényl)-2 méthyl]méthylamino]-2 éthyl]-5 dihydro-2,3 5H-benzothiazépine-1,5 one-4.

Dans un ballon de 250 ml on introduit 4 g (0,00915 mole) de (+)-cis-(2S,3S)-acétyloxy-3 (méthoxy-4 phényl)-2 (méthylamino-2 éthyl)-5 dihydro-2,3 5H-benzothiazépine-1,5 one-4, 3 g (0,0366 mole) de carbonate de potassium, 2,3 g (0,0137 mole) de α-chloro méthylènedioxy-3,4 méthylbenzène et 100 ml de butanone-2 et on chauffe le mélange au reflux pendant 24 h. On filtre le mélange, on évapore le filtrat et on purifie l'huile résiduelle par chromatographie sur colonne de silice en éluant avec un mélange 97/3 de dichlorométhane/méthanol. On dissout la fraction pure dans 60 ml d'éthanol, on la traite avec 0,76 g d'acide oxalique, on filtre le sel qui précipite et on le recristallise dans l'éthanol. On isole finalement 2,71 g d'oxalate de (+)-cis-acétyloxy-3 (méthoxy-4 phényl)-2 [[N-[(méthylènedioxy-3,4 phényl)-2 méthyl]méthylamino]-2 éthyl]-5 dihydro-2,3 5H-benzothiazépine-1,5 one-4. Point de fusion : 71°C.

Le tableau ci-après illustre les structures chimiques et les propriétés physiques de quelques composés selon l'invention.

Tableau

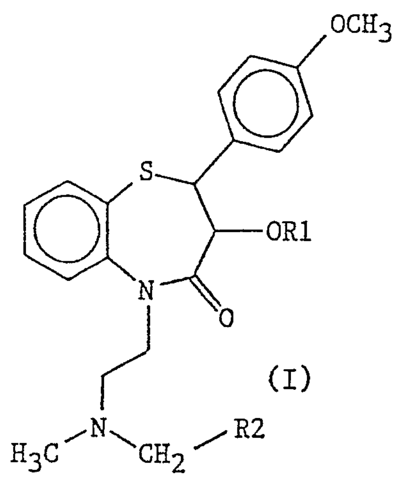

(I)

| N° | R1 | R2 | Sel* | $[\alpha]_D^{20}$ (°) c, MeOH (%) | F(°C) |
|---|---|---|---|---|---|
| 1 | $COCH_3$ | $C_6H_4$-3-$OCH_3$ | ox. | +86,5 (0,5) | 164 |
| 2 | $COCH_3$ | $C_6H_3$-3,4-$(OCH_3)_2$ | ox. | +84,8 (0,5) | 152 |
| 3 | $COCH_3$ | $C_6H_3$-3,4-$OCH_2O$ | ox. | +87,5 (0,5) | 71 |
| 4 | $COCH_3$ | $C_6H_3$-3,5-$(OCH_3)_2$ | ox. | +83,3 (0,5) | 184 |
| 5 | $COCH_3$ | $C_6H_3$-2,5-$(OCH_3)_2$ | ox. | +89,3 (1) | 108 |
| 6 | $COCH_3$ | $C_6H_3$-2,3-$(OCH_3)_2$ | fum. | +73,3 (1) | 123 |
| 7 | $COCH_3$ | $C_6H_3$-3,4-$Cl_2$ | ox. | +82,4 (0,5) | 178 |
| 8 | $COCH_3$ | $C_6H_3$-3,4-$OCH_2CH_2O$ | ox. | +75,9 (1) | 114 |
| 9 | $COCH_3$ | $C_6H_2$-3,4,5-$(OCH_3)_3$ | ox. | +87,6 (0,3) | 162 |

* : ox. oxalate
fum. fumarate

Les composés de l'invention ont été soumis à des essais pharmacologiques montrant leur intérêt en tant que substances thérapeutiques.

Un premier essai a révélé qu'ils sont des antagonistes du calcium. Le protocole expérimental utilisé est une variante de celui utilisé par Godfraind et Kaba (1969), (Blockade or reversal of the contraction induced by calcium and adrenaline in depolarized arterial smooth muscle, Br. J. Pharmac., 36, 549-560).
Les expériences ont été réalisées sur des tronçons d'aorte thoracique de lapin. Les animaux, des "Fauves de Bourgogne" d'un poids moyen de 1,5 kg, sont sacrifiés par dislocation cervicale et exsanguination. L'aorte thoracique est rapidement prélevée et placée dans un milieu de Krebs bicarbonaté oxygéné (95 % $O_2$ + 5 % $CO_2$).
Des tronçons d'aorte de 1 cm de long environ sont préparés et installés dans des cuves à organes de 20 ml contenant de la solution de Krebs bicarbonatée oxygénée (pH 7,4) à 37° C. Deux crochets métalliques en "U" de la longueur des tronçons sont introduits dans la lumière de ceux-ci. L'un des crochets est fixé à la base de la cuve. L'autre, relié à une jauge de contrainte isométrique (Grass FT03), permet l'enregistrement, par l'intermédiaire d'un préamplificateur continu (Grass 7P1) des réponses contractiles des tronçons d'aorte sur un oscillographe à encre (Grass 79B). Cette méthode présente, par rapport aux préparations en spirale ou en anneaux, l'avantage de mieux respecter l'intégrité structurale des vaisseaux et de n'enregistrer que la composante radiale des réponses contractiles qui représente le phénomène intèressant du point de vue fonctionnel (régulation de la pression artérielle). Une tension initiale de 4 g est imposée aux préparations. De la phénoxybenzamine (1 µM) et du propranolol (1 µM) sont ajoutés aux différents milieux de Krebs afin de supprimer les réponses contractiles liées à l'activation des récepteurs α-et β-adrénergiques vasculaires.
Après une heure de stabilisation dans le milieu de Krebs bicarbonaté, la tension imposée aux aortes est ramenée à 2 g. Après une période d'attente de 30 minutes, les préparations sont incubées pendant une dizaine de minutes dans une solution de Krebs bicarbonatée sans calcium en présence d'EDTA (200 µM) et de propranolol (1 uM). Cette solution est alors remplacée par un milieu de Krebs dépolarisant (riche en potassium et appauvri en sodium) sans calcium et contenant du propranolol (1 µM). Après 5 minutes, une concentration unique de 1 mM de calcium est ajoutée à cette solution et une période de stabilisation de 30 minutes est respectée qui permet aux préparations d'atteindre une contraction stable. Ensuite, les composés à tester sont ajoutés au bain, à doses cumulatives, un délai de 30 minutes (temps généralement nécessaire pour l'obtention d'un palier) étant respecté entre deux concentrations, jusqu'à disparition totale de la contraction provoquée par 1 mM de calcium ou bien jusqu'à la concentration maximale de 30 µM de produit. En fin d'expérience, une concentration supramaximale de papavérine (300 µM) est ajoutée afin de déterminer la décontraction maximale possible de chaque préparation.
Les valeurs absolues (en grammes) de la contraction initiale après 1 mM de $CaCl_2$) et de la contraction après les différentes concentrations cumulatives de composés vasodilateurs sont obtenues, pour chaque préparation, par différence avec la contraction minimale observée 30 minutes après l'addition finale de 300 µM de papavérine. Le pourcentage de diminution de la contraction, par rapport à la contraction provoquée par 1 mM de calcium, est calculé pour chaque concentration de composé et chaque préparation et ce pourcentage individuel de décontraction est moyenné X ± S.E.M. Les valeurs moyennes obtenues (pondérées par l'inverse de l'erreur standard à la moyenne), sont analysées à l'aide d'un modèle mathématique de courbe sigmoïde. On calcule la concentration molaire provoquant 50% de décontraction de la réponse au calcium ($CE_{50}$), ou bien son antilogarithme ($pCE_{50}$).
Pour les composés de l'invention les $pCE_{50}$ sont de l'ordre de 5,0 à 7,0.

Les composés de l'invention ont aussi fait l'objet d'un essai d'inhibition de la liaison spécifique du "PAF", facteur d'activation des plaquettes.

Les animaux sont des lapins d'un poids de 2,5 à 3 kg, anesthésiés au pentobarbital sodique (0,25 mg/kg par voie intraveineuse) et maintenus sous respiration artificielle par intubation. On prélève le sang dans l'artère carotide et on le recueille dans des tubes contenant un anticoagulant à base de citrate. On soumet les tubes à une centrifugation à 100g pendant 15 mn, et on dilue le plasma riche en plaquettes avec 2 volumes de tampon à 10 mM de Tris-HCl, pH 7,5, contenant 150 mM de chlorure de sodium et 2 mM d'EDTA (tampon A). Après centrifugation à 1000g pendant 10 mn à 4° C, on lave le culot avec 2 volumes de tampon A, et on effectue une nouvelle centrifugation.
On met le culot riche en plaquettes en suspension dans un tampon glacé (10 mM de Tris-HCl, pH 7,0, contenant 5 mM de chlorure de magnésium et 2 mM d'EDTA), on l'homogénéise et on le centrifuge à 30000g et à 4° C pendant 10 mn. On répète cette opération, on recueille le culot résultant dans le même tampon, on l'homogénéise et on congèle la suspension de membranes dans l'azote liquide. Pour l'essai d'inhibition de la liaison, on dègèle la suspension et on la dilue avec du tampon pour obtenir une teneur d'environ 150 µg de protéine par millilitre.
On fait incuber des quantités aliquotes (30 µg de protéine) de membranes en présence de tampon à 10 mM de Tris-HCl, pH 7,0, contenant 10 mM de chlorure de magnésium et 0,25% (en poids par volume) d'albumine de sérum bovin, pendant 2 h à 25°C, avec 1 nM de PAF tritié ([$^3$H] 1-O-hexadécyl-2-acétyl-sn-glycéryl-3-phosphorylcholine), dans un volume final de 1 ml. On termine l'incubation en recueillant les membranes et en les lavant rapidement avec du tampon glacé sur des filtres Whatman, en utilisant un collecteur de cellules Skatron connecté à une pompe à vide. On sèche les filtres et on les dose par spectrométrie scintigraphique. On définit la liaison spécifique par la différence de radioactivité des membranes observée en l'absence et en la présence de 1 µM de PAF tritié.

Les essais d'inhibition de la liaison sont effectués dans les conditions décrites, en présence de différentes concentrations de composés à tester. On détermine ensuite la concen tration $CI_{50}$ pour chaque composé (concentration qui inhibe de 50% la liaison spécifique) en traçant la courbe d'inhibition selon la méthode des moindres carrés.
Les $CI_{50}$ des composés de l'invention, dans cet essai, se situent entre 3 et 8 µM.

Enfin les composés de l'invention ont fait l'objet d'un essai d'inhibition de l'agrégation des plaquettes du sang induite par le "PAF-acéther" (1-O-($C_{16}$-$C_{18}$-alkyl)-2-acétyl-sn-glycéryl-3-phosphorylcholine).
L'essai est effectué selon la méthode de Born (J. Physiol., (1963), 168, 178-195) sur des plaquettes de lapin. On prélève le sang par ponction cardiaque, et on le recueille sur du citrate trisodique à 3,8% dans les proportions de 1 volume de solution anti-coagulante pour 9 volumes de sang. On effectue ensuite une centrifugation à 250g pendant 10 mn, on prélève le plasma riche en plaquettes et on procède à la numération des plaquettes.
On soumet le culot à une nouvelle centrifugation, à 3600g pendant 15 mn, de façon à obtenir du plasma pauvre en plaquettes, et on dilue le plasma riche en plaquettes au moyen de plasma pauvre en plaquettes, pour obtenir une suspension contenant de 200000 à 300000 plaquettes par microlitre.
On provoque l'agrégation in vitro au moyen de PAF-acéther à la concentration maximale nécessaire pour obtenir une réponse réversible maximale (normalement entre 1,5 et 3,3 ng/ml). On enregistre les variations de densité optique au moyen d'un agrégomètre (300 µl de plasma riche en plaquettes par cuve, 1100 t/mn à 37°C) jusqu'au dépassement de l'agrégation maximale.
Pour les essais, on dissout les composés de l'invention dans du diméthylsulfoxyde et on les fait incuber pendant 2 mn à 37°C avant l'addition du PAF-acéther.
L'action anti-agrégante des composés s'exprime par la concentration $CI_{50}$, concentration qui inhibe de 50% l'agrégation provoquée par le PAF-acéther.
Les $CI_{50}$ des composés de l'invention, dans cet essai, se situent entre 1 et 10 µM.

Les résultats des essais pharmacologiques montrent que les composés de l'invention sont des antagonistes du calcium et peuvent, à ce titre, être utilisés pour le traitement de diverses affections pour lesquelles ce type d'agents est indiqué.
C'est ainsi qu'en particulier ils peuvent être utilisés en médecine cardiovasculaire pour le traitement d'affections nécessitant des modulateurs des mouvements transmembranaires et intracellulaires du calcium, tout spécialement l'hypertension, l'angor et l'arythmie cardiaque.
Ils présentent en outre des effets antiathérogènes, antiagrégants plaquettaires, anti-ischémiques cardiaques, anti-ischémiques cérébraux, antimigraineux, antiépileptiques, antiasthmatiques et antiulcéreux.
Dans le domaine cardiovasculaire ils peuvent être utilisés seuls ou associés à d'autres substances actives connues telles que les diurétiques, les β-bloquants, les inhibiteurs de l'enzyme de conversion de l'angiotensine, les antagonistes des récepteurs $\alpha_1$.
Combinés avec des agents destinés à potentialiser leurs effets ou à diminuer leur toxicité, ils peuvent être également indiqués pour le traitement du cancer ou dans les transplantations.

Les composés de l'invention peuvent être présentés sous toutes formes appropriées à l'administration orale ou parentérale, en association avec des excipients connus, par exemple sous forme de comprimés, gélules, dragées, capsules, solutions ou suspensions buvables ou injectables.
La posologie journalière peut aller par exemple de 30 à 300 mg par la voie orale et de 25 à 100 mg par voie parentérale.

**Revendications**

1. Composés, sous forme de diastéréiosomères purs ou de leurs mélanges, répondant à la formule générale (I)

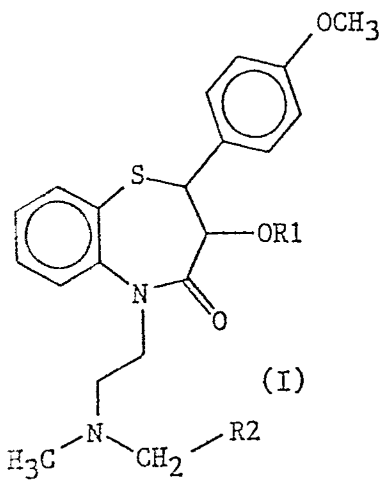

(I)

dans laquelle
R1 représente un atome d'hydrogène ou un groupe alcanoyle en $C_2$-$C_4$,
R2 représente un groupe phényle portant éventuellement de 1 à 3 substituants choisis parmi les atomes d'halogène et les groupes méthylènedioxy, éthylènedioxy, et alcoxy en $C_{1-4}$, ainsi que leurs sels d'addition à des acides acceptables en pharmacologie.

2. Procédé de préparation des composés selon la revendication 1, caractérisé en ce qu'on fait réagir une benzothiazépinone de formule (VI)

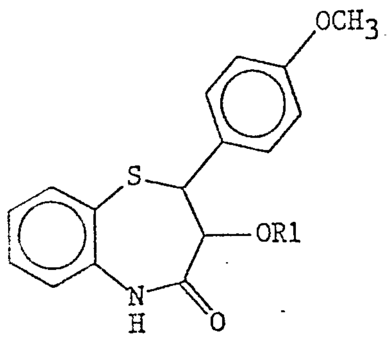

(VI)

(dans laquelle R1 est tel que défini dans la revendication 1) avec une amine halogénée de formule (V)

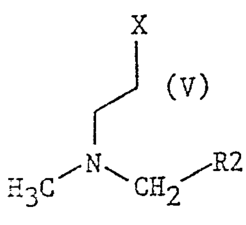

(V)

(dans laquelle R2 est tel que défini dans la revendication 1 et X représente un atome d'halogène).

3. Médicament caractérisé en ce qu'il consiste en un composé selon la revendication 1.

4. Composition pharmaceutique, caractérisée en ce qu'elle contient un composé selon la revendications 1, associé à un excipient pharmaceutique.

**Revendication pour les Etats contractants suivants: ES, GR**

Procédé de préparation de composés répondant à la formule générale (I)

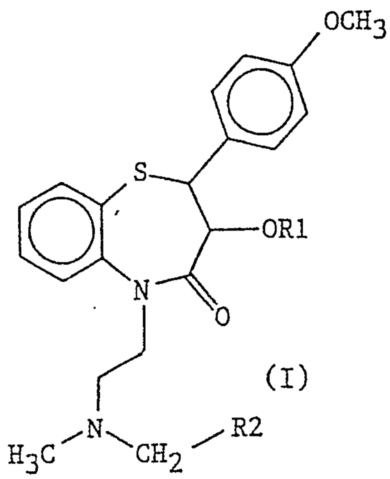

(I)

dans laquelle
R1 représente un atome d'hydrogène ou un groupe alcanoyle en $C_2$-$C_4$,
R2 représente un groupe phényle portant èventuellement de 1 à 3 substituants choisis parmi les atomes d'halogène et les groupes méthylènedioxy, éthylènedioxy, et alcoxy en $C_1$-$C_4$, procédé caractérisé en ce qu'on fait réagir une benzothiazépinone de formule (VI)

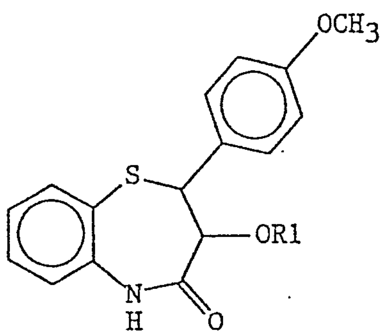

(VI)

(dans laquelle R1 est tel que défini ci-dessus) avec une amine halogénée de formule (V)

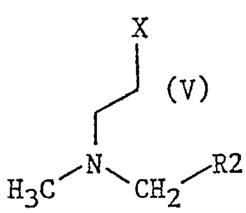

(V)

(dans laquelle R2 est tel que défini ci-dessus et X représente un atome d'halogène).

Office européen des brevets

# RAPPORT DE RECHERCHE EUROPEENNE

Numero de la demande

EP 88 40 3078

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | "THE MERCK INDEX - An encyclopedia of chemicals, drugs, and biologicals", édition 10, 1983, page 466, Merck & Co., Inc., Rahway, New York, US<br>* Page 466, no. 3189: "Diltiazem" * | 1-4 | C 07 D 281/10<br>C 07 D 417/12<br>A 61 K 31/55 |
| Y | EP-A-0 065 229 (K. THOMAE)<br>* En entier * | 1-4 | |
| P,X | EP-A-0 256 888 (McNEILAB)<br>* En entier * | 1-4 | |

DOMAINES TECHNIQUES RECHERCHES (Int. Cl.4)

C 07 D 281/00
C 07 D 417/00
A 61 K 31/00

Le présent rapport a été établi pour toutes les revendications

| Lieu de la recherche | Date d'achèvement de la recherche | Examinateur |
|---|---|---|
| LA HAYE | 08-03-1989 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)